Europäisches Patentamt

European Patent Office

Office européen des brevets

(11) Publication number: **0 238 332**
**A2**

# EUROPEAN PATENT APPLICATION

(21) Application number: **87302354.3**

(22) Date of filing: **18.03.87**

(51) Int. Cl.⁴: **C 12 Q 1/68**

(30) Priority: **19.03.86 US 841155**

(43) Date of publication of application:
**23.09.87 Bulletin 87/39**

(84) Designated Contracting States:
**AT BE CH DE FR GB IT LI LU NL SE**

(71) Applicant: **CETUS CORPORATION**
**1400 Fifty-Third Street**
**Emeryville California 94608 (US)**

(72) Inventor: **Goodson, Robert James**
**723 Cornell Avenue**
**Albany California 94706 (US)**

**Sheridan, Patrick James**
**2008 Horne Street**
**San Leandro California 94578 (US)**

(74) Representative: **Bizley, Richard Edward et al**
**BOULT, WADE & TENNANT 27 Furnival Street**
**London EC4A 1PQ (GB)**

(54) Liquid hybridization method and kit for detecting the presence of nucleic acid sequences in samples.

(57) A particular nucleic acid sequence present in a test sample can be detected in an assay wherein extracted nucleic acids in the test sample are hybridized in the presence of a molar excess of at least two oligonucleotide probes, one of which is attached to detectable label moiety(ies) and the other of which is attached to a binding moiety different from the label moiety(ies) and specifically recognizing a complementary binding molecule. The probes are complementary to different non-overlapping portions of the same strand of the nucleic acid sequence to be detected. After hybridization, the mixture is contacted with a solid support carrying the complementary binding molecule specifically recognized by the binding moiety, and the support is washed and exposed to detection means.

EP 0 238 332 A2

**Description**

LIQUID HYBRIDIZATION METHOD AND KIT FOR DETECTING THE PRESENCE OF NUCLEIC ACID
SEQUENCES IN SAMPLES

The present invention relates to a method and kit for detecting particular nucleotide sequences which are suspected of being contained in a test sample containing the nucleic acids from one or more organisms. In particular, the method involves using at least two oligonucleotide probes which are complementary to mutually exclusive portions of the same target nucleic acid in a liquid hybridization format to anneal the probes to the target if it is present, and detecting the presence of the target by immobilization of the two-probe target sandwich by one of the probes and subsequent detection of the other probe.

Currently employed methods for DNA probe-based assays include Southern blot and dot blot hybridizations. Both of these methods rely on liquid-solid hybridizations, which are slow and inefficient and are not easily automated. The Southern blot protocol has the further disadvantage of inefficient transfer from gel to paper.

Another type of liquid-solid hybridization is described by U.S. Patent No. 4,358,535 (Falkow), Ranki et al. (1983) Gene, 21:7785, and European Patent Publication No. 130,515. U.S. 4,358,535 describes in general the method using a single probe to detect an infectious organism. A sandwich system utilizing two mutually exclusive probes to the same target is used to detect adenovirus DNA in the Ranki et al. article and to detect DNA sequences containing a restriction site of interes, preferably sickle cell anemia, in the EP publication. In both cases, one of the probes is preferably bound to a solid support, and the other is bound to a signal-generating label. The two probes are contacted under hybridization conditions with the extracted and denatured DNA sample, and the unhybridized and singly hybridized probes are removed. The detection of the dual hybridization product indicates whether the target is present or absent. This method can be run on relatively crude samples and can remove potentially interfering debris by wash steps prior to detection, but has the drawbacks of solid-liquid phase hybridization kinetics and difficulty in automation.

In contrast to liquid-solid hybridization, liquid hybridization, in which the probe and target are contacted in a homogeneous solution, has very rapid kinetics and improved sensitivity due to greater access of the probe to the target sequences. For example, Gen-Probe Inc. uses a liquid hybridization hydroxyapatite method to detect DNA sequences. The main disadvantage of this system is that it relies on adsorptive discrimination of double-stranded from single-stranded DNA sequences rather than sequence-specific separation of hybrid from excess probe. This former separation is imperfect because the vast excess of probe added relative to the target sequence will inevitably cause some reporter probe to be bound along with the hybrids of interest. The contaminating reporter probe may represent a serious background problem.

Another potential advantage of liquid hybridization is that a generalized solid support can work for a multitude of targets if the support-binding probes are labeled with the same reagent.

European Patent Publication Nos. 70,685 and 70,687 also describe liquid hybridization. The former patent application proposes a hybridization assay technique which dispenses with the need to separate physically the hybridized from unhybridized probe. The proposal is to employ a pair of probes which hybridize to contiguous regions on a polynucleotide sequence of interest and to label one probe with a chemiluminescent catalyst such as peroxidase and the other with an absorber molecule for the chemiluminescent emission. The catalyst and absorber labels must be situated near the contiguous terminal ends of the respective probes such that upon hybridization the chemiluminescent emission is quenched by energy transfer to the absorber molecule. The assay depends on controllable synthesis of two critical probe reagents so that the respective labels are brought into a quenching orientation upon hybridization to the sample nucleic acid and without affecting the probes' affinity for hybridization. Also, because no solid support is present, there is no provision for wash steps, and this may lead to non-specific increases or decreases of the signal depending on assay format and/or the debris in question.

It is known that nucleic acids can be chemically modified, as by using N-acetoxy-N-2-acetylaminofluorene or its 7-iododerivative, for immunoprecipitating complementary or partially complementary sequences and for detecting complementary DNA nonisotopically. Such method, described by Tchen et al. (1984) PNAS, 81:3466-3470, does not, however, describe use of a two-probe sandwich ummunoassay system or of probes in a liquid hybridization assay.

There is a need in the art for a simple method which utilizes the rapid kinetics of liquid hybridization, and also allows the hybridized material to be washed to remove debris from crude samples.

Accordingly, the present invention provides a liquid hybridization sandwich assay for detecting nucleic acid sequences using two probes which is both rapid and sensitive, can be read visually or on a simple plate reader, and may be readily automated.

More specifically, the present invention provides a liquid hybridization method for detecting whether a particular target nucleic acid sequence suspected of being contained in a test sample of one or more nucleic acids is contained therein comprising the steps of:

(a) extracting the nucleic acids in the test sample;

(b) separating any extracted double-stranded nucleic acids into single strands;

(c) exposing the single-stranded nucleic acids to a molar excess of at least two oligonucleotide probes capable of hybridizing with the target sequence, at least one of which probes is a reporter probe linked to the same or different label moieties capable of being detected, and at least one of which probes is a support-binding probe linked to a binding moiety which specifically recognizes a complementary binding molecule and which is different from the label moiety or moieties used for the reporter probe or probes, wherein all of said probes are complementary to different non-overlapping regions of the same strand of said nucleic acid sequence to be detected;

(d) subjecting the mixture from step (c) to hybridization conditions favoring hybridization of said probes to said sequence;

(e) contacting the mixture of step (d) with a solid support phase which carries said complementary binding molecule specifically recognized by the binding moiety of said support-binding probe;

(f) washing the solid support phase to remove any unhybridized nucleic acids and cellular debris from the support phase; and

(g) detecting the presence or absence of said sequence by exposing the solid support to one or more means for detecting the label moiety or moieties of said reporter probe or probes.

Preferably the nucleic acids are DNA, the label moiety for the reporter probe(s) is biotin, and the binding moiety for the support-binding probe is a hapten, to which antibodies on the solid support will specifically bind.

In another aspect of the invention, a liquid hybridization assay kit is provided for determining whether a particular nucleic acid sequence suspected of being contained in a test sample on one or more nucleic acids is contained therein comprising:

(a) at least one oligonucleotide reporter probe which is linked to a label moiety capable of being detected and which is complementary to a first region of one strand of the nucleic acid sequence to be detected;

(b) an oligonucleotide support-binding probe which is linked to a binding moiety which is different from said first label moiety and which specifically recognizes a complementary binding molecule, wherein the support-binding probe is complementary to a second region of the same strand of said sequence which region does not overlap the first region of said sequence, and

(c) a solid support carrying said complementary binding molecule which is specifically recognized by the binding moiety of the support-binding probe.

The method and kit herein avoid the disadvantages of solely liquid-solid hybridizations and solely liquid hybridizations and combine the advantages of both. Thus, hybridizations can be done on crude samples as with liquid-solid hybridizations, which allow for washing the solid support, but also have the rapid kinetics associated with liquid hybridizations. The use of two probes will ensure the accuracy of the detecting system. Furthermore, the method herein does not require expensive equipment to read the assay results, because they can be read visually for a yes-no answer or on a simple plate reader for quantitation. The reaction products can be photographed for permanent records.

The term "liquid hybridization" as used herein refers to a hybridization of nucleic acid probes and target sequence which takes place in a liquid medium without the presence of any solid support attached either to the probe(s) or to the target sequence.

The term "extracting" as used herein refers to the process of taking nucleic acids from the cells in the test sample and making the nucleic acids available to enzymes and probes. Extraction may include, for example, disruption of cells, and separating the nucleic acids from the cellular debris.

The term "oligonucleotide" as used herein to describe the probe refers to a molecule comprised of two or more deoxyribonucleotides or ribonucleotides, preferably more than three. Its exact size will depend on many factors, mainly the target nucleic acid sequence suspected of being present in the test sample.

The term "test sample" as used herein refers to a sample containing one or more nucleic acids, and may be purified or non-purified, provided it contains or is suspected of containing the specific nucleic acid sequence desired. The nucleic acid sequence to be detected may be, for example, DNA or RNA, including messenger RNA, and the DNA or RNA may be single or double stranded. In addition, a DNA-RNA hybrid which contains one strand of each may be utilized. A mixture of any of these nucleic acids may also be employed. Preferably herein the nucleic acids are double-stranded and are DNA. The specific nucleic acid sequence to be detected may be only a fraction of a larger molecule or can constitute the entire nucleic acid. The test sample may be crude, in that the nucleic acid sequence suspected of being contained in the sample is a minor fraction of a complex mixture and/or the sample consists of an unpurified mixture of several different types of nucleic acids. For example, the sequence may constitute a portion of the ß-globin gene contained in whole human DNA or a portion of nucleic acid sequence from a particular microorganism which represents only a very minor fraction of a particular biological sample. The test sample may be obtained from any source, for example, from plasmids such as pBR322, from cloned DNA or RNA, or from natural DNA or RNA from any source (cells, tissue, and body fluid), including bacteria, yeast, viruses, and higher organisms such as plants or animals. Examples of body fluids include blood serum, cerebrospinal fluid, amniotic fluid, tears, saliva, urine, sputum, and the like. Cells and viral and biological tissue extract material may also be test samples.

In the first step of the process herein, the test sample is treated to extract the nucleic acids contained therein. This may be done by any appropriate means. For example, RNA or DNA may be extracted from body fluids such as blood or urine, tissue material such as chorionic villi, or cells such as amniotic cells by a variety of techniques such as that described by Maniatis et al.,Molecular Cloning (1982), 280-281.

After the first step, the nucleic acids may optionally be reduced in size by sonication or contacted with an appropriate restriction enzyme not recognizing a site within the target sequence to obtain a target sequence of sufficient length to have two or more regions of non-contiguous homology. The restriction fragments may be less viscous, less susceptible to shearing in the middle of the hybridization regions, and faster to denature and hybridize. The enzyme must be destroyed (as by heat or chemicals) before exposure to the probes, if either of the probes contains the restriction site.

If the nucleic acids in the test sample are double stranded, it is necessary to separate the strands thereof before the hybridization step. This strand separation, or denaturation, can be accomplished by any suitable method, including physical, chemical or enzymatic means. One physical method of separating the strands of the nucleic acid(s) involves heating the nucleic acid(s) until it/they are completely ($>99\%$) denatured. Typical heat denaturation may involve temperatures ranging from about 80 to 105°C for times ranging from about one to ten minutes. Quenching of the denaturation is accomplished by rapid cooling. Strand separation of DNA may also be accomplished by adding base to the DNA generally at 0.1 to 1 N NaOH, followed by neutralizing to pH 6-8-5. Base cannot be added to RNA as it will degrade the RNA.

After any neutralization step is carried out, the single-stranded nucleic acid(s) are hybridized to two types of oligonucleotide probes, one type designated hereinafter as the reporter probe, which may be one or more probes, and the other type desgnated hereinafter as the support-binding probe.

The reporter probe or probes are linked to the same or different label moieties capable of being detected. The linkage is as strong as or stronger than nucleic acid hybridization interactions and generally is a covalent linkage. Preferably more than one reporter probe are employed, and the same label moiety is used for each so that the signal is enhanced. The label moiety employed for each probe is a moiety which is capable of producing a detectable signal, e.g., an enzyme which can be detected in small quantities by a process which generates a signal. Examples of suitable such detection means include spectroscopic or photochemical means, e.g., absorbance, fluorescence or chemiluminescence, or radioactive, biochemical, immunochemical or chemical means such as changes in physical, biochemical, immunochemical or chemical properties on contact with a detector analysis compound or reaction with a polypeptide or polypeptide/enzyme mixture to form a detectable complex. Thus, in this context the term "label" is intended to include both moieties which may be detected directly, such as radioisotopes or fluorophores, and reactive moieties which are detected indirectly via a reaction which forms between the label moiety and another moiety which is capable of producing a detectable product, such as boitin, which reacts with an avidin- or streptavidin-enzyme conjugate, an enzyme, which reacts with substrate to form a product detectable by colorometric or fluorometric means, or a hapten, which reacts with an antibody specific therefor labeled with a detectable moiety.

The preferred hybridization reporter probe(s) herein is/are nonradioactively labeled, to avoid the disadvantages associated with radioactivity analysis, and is/are resistant to the hybridization conditions. The preferred detection means herein are spectroscopy or photochemistry or the formation of a detectable complex between the label moiety and a polypeptide, lectin, or antibody linked to an entity capable of generating a detectable change, including enzymes, such as, e.g., alkaline phosphatase or horseradish peroxidase, which have chromogenic or fluorogenic substrates, or luciferase, which can generate luminescence. Examples of such complexes are as follows: If the label moiety is biotin, a complex of the polypeptide avidin with an enzyme such as horseradish peroxidase or alkaline phosphatase or a conjugate of avidin and chemi- or bioluminescent labels may be employed as the detection means. If avidin is the label moiety, then biotin can be used in conjuction with a chemiluminescent reagent, bioluminescent reagent, or enzyme. If a hapten (which is immunogenic only when attached to a suitable carrier) is used as the label moiety, such as, e.g., fluorescein, dinitrophenol, or N-acetoxy-N-2-acetylaminofluorene, then appropriate detectable antibodies can be used as the detection means. For example, if dinitrophenol is employed as label moiety, a suitable antibody would include, e.g, monoclonal or polyclonal antidinitrophenol antibodies. The antibodies may be directly detected by a label attached thereto such as, e.g., an enzyme, a chemiluminescent reagent, a bioluminescent reagent, a fluorophore, or Staph A protein labeled with detectable molecules. Alternatively, the antibodies may be detected indirectly by adding a second labeled antibody specific to the first antibody. If carbohydrates are used as label moieties, labeled lectins may be employed as the detection means. The most preferred label moiety herein for the reporter probe or probe(s) is a hapten or biotin, where the most preferred detection means for biotin is an avidin- or streptavidin-enzyme conjugate.

The support-binding probe is a single probe which is linked to a moiety (hereinafter called binding moiety), which is capable of attaching to a solid support phase and which is different from the label moiety or moieties used in conjuction with the reporter probe(s). The attachment of probe to solid support phase is stronger than nucleic acid hybridization interactions. The binding moiety employed for this purpose may be chosen from any of those described above with respect to the reporter probe(s) which specifically recognize a complementary binding molecule, such as, e.g., a polypeptide, lective or antibody, provided it is not the same as that actually used for the reporter probe(s). The binding moiety for the support-binding probe must be different because the reporter moiety must bind to the solid support only by indirect association with the binding moiety.

The greater the difference between the binding moiety and the label moiety, the better the sensitivity and specificity to prevent false negative and positive results, respectively, in the diagnostic tests. This difference is measured by the extent of cross-reactivity between the two types of moieties. Cross-reactivity can be measured by the gel immunodiffusion test of Ouchterlony, by competitive radioimmunoassay, or by equilibrium dialysis.

The stronger the interaction between the binding moiety and the complementary binding molecule, the lower the detection limit is likely to be. Binding strength can be expressed quantitatively with the dissociation constant parameter, or Kd. The lower the Kd, the stronger the binding. For example, one of the strongest known binding reactions, that between avidin and biotin, has a Kd near $10^{-15}$ M. On the other hand, many antibody-antigen reactions have Kd values on the order of $10^{-6}$-$10^{-7}$ M where the detection limit may be unacceptably high. Preferably, the Kd for the binding reaction holding the support-binding probe to the support is less than $10^{-7}$ M. Most preferably, the Kd is less than $10^{-10}$ M. Examples of haptens or antigens known to elicit antibodies with such high affinity include fluorescein, gastrin, digoxin, HbS, insulin, and dinitrophenol.

The complementary binding molecule for the binding moiety of the probe will be coated on or attached to the solid support, which is exposed to the mixture after hybridization. For example, if the binding moiety for the support-binding probe is biotin, a solid support to which has been attached avidin or streptavidin is later contacted with the hybridized mixture. If the binding moiety is a hapten such as fluorescein or dinitrophenol, the solid support carries antibodies specific to the hapten. Preferably, the binding moiety on the support-binding probe is a hapten, and most preferably fluorescein because of its ability tc elicit antibodies of very low Kd value.

The probes themselves are oligonucleotide probes, their particular composition being dependent on the particular nucleic acid sequence to be detected. Oligodeoxyribonucleotide and oligoribonucleotide probes can be used to detect both RNA and DNA sequence. Preferably the probes are oligodeoxyribonucleotides. In addition, the probes must be chosen so that they are each capable of hybridizing to the same strand and complementary to different non-overlapping regions of the nucleic acid sequence to be detected. This is essential to the assay herein; otherwise the probes might compete with one another to lower assay sensitivity.

The probes may also be allele-specific probes which encompass a region of the target nucleic acid sequence spanning a base pair variation to be detected and which are specific for the sequence variation being detected. For example, if it is desired to detect whether a sample contains the mutation for sichle cell anemia, one probe will be prepared which is 5' to a diagnostic restriction site and the other probe(s) will be prepared which are 3' to the same restriction site on the same strand. These probes are described in more detail by Conner et al., PNAS (USA), 80:278 (1983).

The exact lengths of the probes will depend on several factors. If they are too short, the probes may hybridize to nucleic acid sequences other than the targeted one; the shorter the sequence, the more frequently its complement will be found in the nucleic acids of an organism. In addition, base pairing of short sequences will tend to be energetically weak, restricting the detection limit in the same fashion as weak binding reaction of the support-binding probe to the support. On the other hand, very long probes will show slow kinetics of hybridization, and have an increased probability of containing self-complementary sequences which can form internally base-paired secondary structure. Such self-hybridization would compete for hybridization with target nucleic acid sequences, decreasing assay sensitivity. The probe may be longer than the hybridizing region of the target molecule, such that binding moieties are attached to excess.

The probes may be prepared by restriction digestion, by subcloning, or by synthetic production using techniques well known in the art. Thus, for example, a nucleic acid having a sequence substantially identical to the sequence to be detected may be cloned and then digested with one or more restriction enzymes, depending on how many probes are to be prepared and how many convenient restriction sites are present in the sequence, which enzyme(s) will cut the sequence at the appropriate restriction site(s), leaving fragments of appropriate base pair length to be useful as probes. These resulting fragments are then separated from the restriction digest by any suitable means, such as electrophoresis. They may be separated from each other by, for example, subcloning. Thus, the restriction fragments are cloned randomly into an appropriate vector at a complementary restriction site to the fragments generated. The vector and fragments are allowed to ligate and the new molecule is used to transform an appropriate host. The proper clones are selected based on either a selectable expressed marker or upon DNA analysis via electrophoresis based on size.

The hybridizing portion of the probe may be cloned into M13 to obtain single-stranded nucleic acids as described by Messing, J. (1981) Third Cleveland Symposium on Macromolecules:Recombinant DNA, ed. A. Walton, Elsevier, Amsterdam, 143-153. Also, a "gapped circle" may be utilized as a probe, as described by Courage-Tebbe et al., Biochim. Biophys. Acta, 697:1-5 (1982), where the hybridizing region is the only single-stranded portion of the molecule.

In another embodiment, the probes are obtained by using commonly known recombinant DNA techniques to subclone each of the desired oligonucleotide fragments in separate host vector systems such as. e.g., pBR322 and pSC101, a variant plasmid of pBR322 with additional desirable unique restriction sites for insertions, which was deposited with the American Type Culture Collection under ATCC No. 37,032 and is described in U.S. Patent Nos. 4,237,224 and 4,339,538.

In a third embodiment, the probes may be produced synthetically, such as by the phosphotriester or phosphodiester methods described by Narang et al., Meth. Enzymol. (1979) 68:90 and Brown et al., Meth. Enzymol. (1979) 68:109, respectively, or automated embodiments thereof. In one such automated embodiment diethylphosphoramidites are used as starting materials and may be synthesized as desribed by Beaucage et al., Tetrahedron Letters (1981) 22:1859-1862. One method for synthesizing oligonucleotides on a modified solid support is described in U.S. Patent No. 4,458,066.

In a fourth embodiment, the probes may be prepared by amplifying a nucleic acid sequence as described in Saiki et al., Science, 230, 1530-1534 (1985).

After the probes are prepared, they are attached to their respective label and binding moieties by any suitable attachment technique, preferably covalent. The probe may be attached to the label using psoralen as intercalation agent as described by U.S. Patent No. 4,582,789.

Furthermore , the well known nick translation method for incorporating labels as described by Ward in European Patent Application 63,879 may be employed. Also, RNA may be labeled with fluorochrom as described by Bauman et al., J. Histochem. Cytochem., 29:227-237 (1981). Also, guanine residues in RNA or DNA may be chemically modified using N-acetoxy-N-2-acetylaminofluorene (AAF) or its 7-iodo derivative (AAIF) according to Tchen et al., PNAS (USA) 81:3466-3470 (1984). Also, Poirier et al., Nature, 270:186-188 (1977) describes DNA adducts of acetylaminofluorene.

In the actual hybridization, the single-stranded nucleic acid(s), which may be in admixture with cell debris, etc., is/are added to a hybridization solution which will depend on the specific hybridization conditions employed. Such solution may consist of 100% water or up to about 80 volume percent, preferably 20-60%, of an inert polar organic solvent such as formamide containing various salts, Ficoll, serum albumin, polyvinylpyrrolidone and a buffer at pH 6-9. Details of liquid hybridization conditions which can be employed herein are provided by Wetmur, Ann. Rev. Biophys. Bioeng., 5:337-361 (1976) and by Gillespie, Recent Adv. Cancer Res: Cell Biol. Mol. Biol., Tumor Virol., 1:139-152 (1977). The probes are then added to this solution in molar excess over stoichiometric amounts relative to the moles of test sample, to enhance the rate of binding of the probes to the nucleic acid sequence. The exact extent of molar excess of the probes in the solution will depend, for example, on the label moieties attached to the probes, the number of binding sites on the solid support, the particular sequence to be detected, and the hybridization conditions. For example, the support-binding probe should not be present in excess over the number of binding sites on the support to avoid reduced sensitivity. Generally, the probes are present in a 10,000 to $10^6$ molar excess, more preferably 100,00 to $1 \times 10^6$ molar excess.

The hybridization conditions employed may have varying degrees of stringency depending on the ultimate goal desired. Stringency is affected by temperature, probe length, ionic strength, etc. Controlling the concentration of the inert organic solvent in the solution over the range of 20 to 50 volume percent will alter the polarity and thus the stringency of the hybridization. Generally the hybridization will occur at about 25 to 75°C, preferably 35 to 65°C, for 0.25 to 20 hours, preferably 1.5-5.0 hours, these conditions being dependent mainly on the relative and absolute concentrations of the specific probes and test sample employed, as well as the concentrations of other ingredients in the medium. One skilled in the art of hybridization will be able to make the appropriate adjustments in conditions. The greater the stringency of the conditions, the greater the required complementarity for hybridization between the probes and the nucleic acid sequence to be detected.

Prior to contact of the hybridization mixture with the solid support, the solid support is preferably treated such that the probes do not bind non-specifically to the support. Examples of blocking agents which can be used to treat the solid support include, e.g., phosphate buffered saline with bovine serum albumin, non-ionic detergents, polyanions, and herring sperm DNA.

After the hybridization is complete, the hybridization mixture is contacted with a solid support which carries (by coating, adsorption or attachment) a binding molecule which is complementary to, and specifically recognized by, the binding moiety of the support-binding probe. This molecule must not cross-react with any other compounds present in the hybridization mixture. Examples of suitable such binding molecules include polypeptides, lectins, or antibodies, depending on the binding moiety employed. Examples of solid supports include microtiter wells, polystyrene beads, polyacrylic beads, agarose beads, nylon beads, derivatized paper such as nitrocellulose paper, water-insoluble porous filters such as, e.g., nitrocellulose or a nylon membrane, and other materials which will be inert to the reaction medium and to the material which is carried on the surface therof. The binding molecule may be adsorbed onto or covalently linked to the support, but for sensitivity reasons is preferably covalently linked thereto. The conditions employed will be suitable for attachment of the complementary binding molecule. One method for this attachment is described by Wood et al., J. Clin. Chem. Clin. Biochem., 21:789-797 (1983).

In the next step of the process herein the solid support is washed to remove any unhybridized nucleic acids such as reporter probes and any remaining cell debris, such as proteins, lipids, mucopolysaccharides, etc., from the test sample. If the support is a filter, a washing solution such as 0.1 to 1% by volume of one or more ionic detergents or combinations of salt(s) and detergents is applied thereto for a certain contact time and removed. If the support is beads, the hybridization mixture containing the beads may be filtered to remove the liquid and the beads contacted with the washing solution, or the fluid may be removed by an automatic liquid handling instrument such as that described in U.S. Patent No. 4,478,094. If the support is a microtiter well, the liquid is removed and the walls of the well are washed with the solution. The washing solution will vary mainly with the types of label moieties and nucleic acids involved, and the degree of stringency required, but typically will consist of salts such as sodium chloride and sodium citrate in combination with a non-ionic detergent such as Tween 20 or Triton x100. The contact time for washing will vary mainly with the type of solution, but generally ranges from about five minutes to three hours or more. The washed support on which the sample is deposited may then be rinsed at room temperature with a dilute salt solution (such as sodium citrate-sodium chloride) if necessary to disrupt non-specific interactions before it is subjected to detection means.

In the final step of the process herein the label moiety(ies) on the reporter probe(s) is/are detected by exposing the washed and preferably blocked solid support to one or more detection means for the label moiety(ies). If the moieties are detected, then hybridization has occurred between the target single-stranded nucleic acid and both kinds of probes, and the nucleic acid sequence suspected of being contained in the sample is, in fact, in the sample. If more than one type of label moiety is utilized, the order of addition of the detection means for each will depend mainly on the types of detection means and label moieties employed. The detection means also may be added simultaneously, if appropriate. Suitable detection means are those described above in conjuction with the discussion of the label moieties for the reporter probe(s) and include radioactive, spectroscopic, photochemical, immunochemical, biochemical or chemical means. Immunochemical means include antibodies which are capable of forming a complex with the reporter probe(s) under suitable conditions, biochemical means include polypeptides or lectins capable of forming a complex with the reporter probe(s) under the appropriate conditions, and photochemical means include ultraviolet or visible light to stimulate fluorescence. In addition, the detection means may consist of a combination of an absorber-emitter moiety and a chemi luminescent catalyst in sufficiently close proximity to each other to permit non-radiative energy transfer, in conjunction with chemiluminescent reagents suitable for inducing a light response in the presence of the chemiluminescent catalyst, as described in European Patent Publication Nos. 70,686 and 70,685. The antibody, polypeptide or lectin must be capable of detection or include a moiety which can be detected when the complex is formed. Examples of such includable detectable moieties include fluorescent dyes, electrondense reagents, or an enzyme capable of depositing an insoluble reaction product or bieng detected spectrophotometrically or fluorometrically. Usually the antibody, polypeptide or lectin will be coupled to an enzyme which will react with a chromogenic substrate. If, as with the use of enzymes, the detection means is capable only of indirect detection, any free detection means is washed away from the solid support and the remaining components such as substrates for enzymes are added to visualize the bound detection system and read the results. For example, using an avidin-enzyme complex to detect hybridized reporter probe labeled with biotin involves washing off the unreacted avidin-enzyme complex, adding a chromogenic substrate for the enzyme, and reading the resulting color change. However, proteins which become luminescent when treated appropriately may also be employed in detecting the labeled probe.

The contact of the hybridization mixture and detection moiety, such as antibodies to an antigen label, is preferably carried out with mixing to ensure sufficient interaction.

The visulization of the results of the test may be accomplished in many ways, depending on the detection means. It does not necessarily require an expensive machine and, if a chromogenic detection system is used, can generally be accomplished visually for yes/no answers or on a simple plate reader for quantitation. The reaction products can be photographed for permanent records.

The process herein can be automated using liquid handling systems such as that described in U.S. Patent No. 4,478,094. Thus, the microtiter wells are coated with the complementary binding meleucles which specifically recognize the binding moiety of the support-binding probe. Once the hybridization has occurred, pneumatically controlled samplers are used to transfer the hybridization mixture to the wells where the fixation takes place. The wells are then automatically drained and washed to remove any material which was not bound to the solid support, including excess reporter probe(s) which has/have not hybridized. A detection means is then used to read the results.

While the method herein has lower background than completely liquid hybridizations because the solid support can be washed to remove excess unwanted material, it can be made more sensitive by amplifying the suspected nucleic acid sequence in the test sample before it is hybridized to the probes by using two oligonucleotide primers to produce extension products thereof in a chain reaction described in Saiki et al., supra. Amplification is particularly useful when the amount of nucleic acid available for analysis is very small, as, for example, in the prenatal diagnosis of sickle cell anemia using DNA from fetal cells, and also is useful if such an analysis is to be done on a small sample where there is fewer than one copy of target nucleic acid per cell.

The method herein may be used to enable detection of specific nucleic acid sequences associated with infectious diseases, genetic disorders or cellular disorders such as cancer.

For purposes of this invention genetic diseases may include specific deletions and/or mutations in genomic DNA from any organism, such as, e.g., sickle cell anemia, cystic fibrosis, $\alpha$-thalassemia, ß-thalassemia, and the like, whether homozygous or heterozygous. Genetic diseases which can be related to the presence or absence of restriction sites, such as sickle cell anemia, may be detected by this method, analogous to the restriction fragment method. For example, normal ß-globin would be cleaved by Ddel or Mstll, with negative results (both probes would hybridize to non-contiguous fragments). The homozygous sickle cell allele would not be cleaved, giving a positive reaction (both probes would hybridize to the same intact target). $\alpha$-Thalessemia can be detected by the absence of a sequence, and ß-thalassemia can be detected by the absence of a sequence, and ß-thalessemia can be detected by the presence of a polymorphic restriction site closely linked to a mutation which causes the disease. Preferably, the gene to be detected is ß-globin.

In the event that the method herein is used to detect a restriction site (as in the case of sickle cell anemia), the probes must be specially designed for the method to work. One of the probes will be 3' to the restriction site of interest and one will be 5' to the restriction site. Neither probe can overlap the restriction site of interest. Both probes hybridize to the same strand.

Preferably neither probe spans a second site of the target which is recognized by the same restriction enzyme as the first site of interest. In any event, both probes cannot so span a second and third site because the results would not be accurate. If one of the probes spans a second sequence representing the same site, the probe which does not span any sequence representing the same site must be the support-binding probe. Otherwise, segments which are extraneous to the results will bind to the solid phase support, competing with

7

the correct fragments.

In this method for detecting a restriction site, the restriction enzyme which recognizes the site of interest is added to the reaction mixture preferably before hybridization, and contacted therewith under essentially the same conditions as used with restriction fragment length polymorphism (see U.S. Patent No. 4,395,486 (Wilson et al.) and Geever et al., PNAS, 78:5081-5085 (1981) to cleave the site if it is present. Then the restriction digest is contacted with the solid support phase and the steps are continued as described above.

Various infectious diseases can be diagnosed using the proper probe to detect the presence in clinical samples of specific DNA sequences characteristic of the causative (infectious) microorganism. These include, e.g., bacteria, such as Salmonella, Neisseria gonorrheae, parastic microorganisms such as Chlamydia and Rickettsiae, viruses such as the hepatitis viruses, and protozoan parasites such as the Plasmodium responsible for malaria. Antibiotic resistances can be screened for such substances as ß-lactamases found on plasmids shared by many pathogenic organisms.

In addition to detecting infectious diseases and pathological abnormalities in the genome of organisms, the process herein can also be used to detect DNA polymorphism which may not be associated with any pathological condition.

The following examples are offered by way of illustration and are not intended to limit the invention in any manner. They represent expected, not actual, results. In these examples all percentages are by weight if for solids and by volume if for liquids, and all temperatures are expressed in degrees Celsius.

EXAMPLE 1

The mutation in the human ß-globin gene responsible for the sickle cell disease is identified using the procedures below.

The assay is performed by preparing two probes from cloned ß-globin DNA. For this purpose, the plasmid pBR328:ßA-1.9 (abbreviated to pBR:ßA) is used as the source of the probes. pßR:ßAwas prepared as follows:

A 1.9 kb BamHI fragment of the normal ß-globin gene was isolated from the cosmid pFCII described by F. Collins et al., PNAS 81, 4894-4898 (1984) and inserted into the BamHI site of pBR328 (Soberon, et al., Gene (1980) 9:287-305). This fragment which encompasses the region that hybridizes to the synthetic 40-mer probe, includes the first and second exons, first intron, and 5' flanking sequences of the gene (Lawn et al., Cell (1978) 15:1157-1174). This clone, pBR:ßA, was deposited on May 25, 1984 with the ATCC under ATCC No. 39,698.

DNA from pBR:ßA is isolated by standard procedures (Maniatis et al. Molecular Cloning (1982). Two separate digestion reactions are set up, using appropriate conditions for digestion as described by the enzyme manufacturer, one of which reactions uses HaeIII and the other of which uses HinfI.

Using standard procedures for preparing blunt-end fragments (described by Maniatis et al., supra), the HinfI digestion fragments are either filled in or digested back prior to ligation into the SmaI site in the poly linker region of M13.

HaeIII digestion fragments are blunt-ended and as such are ready for ligation into the SmaI site of the poly linker region of M13.

The preparation and isolation of M13 are described by J. Messing (1981) Third Cleveland Symposium on Macromolecules:Recombinant DNA, ed. A. Walton, Elsevier, Amsterdam, p, 143-153. M13 DNA is digested with the restriction enzyme SmaI according to the manufacturer's instruction to open a unique cloning site in the poly linker region.

The HaeIII digestion products are blunt-end ligated into the SmaI site of the poly linker region of M13 and the E. coli strain JM103 (BRL) is transformed with this ligation product according to J. Messing, supra.

The blunt-ended HinfI digestion products are blunt-end ligated into the SmaI site of the poly linker region of M13 and the E. coli strain JM103 is transformed in a separate reaction similar to the one described for the HaeIII probe above.

For the HinfI probe, the clones containing a 341 bp Hinfi insert in the proper orientation and spanning positions 2775-3115 from the pBR:ßA donor are selected via a plaque lift and subsequent probing with [32]p-labeled oligomer probes for the sequence desired. (Plaque lifts are described by Benton and Davis, Science, 196:180 (1977).

For the HaeIII probe, the clones containing a 272 bp HaeIII insert in the proper orientation and spanning positions 3181-3456 from the pBR:ßA donor are picked out and probed as described above.

The two M13 clones are grown and the ssDNA is isolated from the culture supernatants as described by Messing, supra.

The HinfI probe is 5' to the diagnostic MstII and DdeI sites and will serve as the support-binding probe.

The HaeIII probe is 3' to the diagnostic MstII and DdeI sites and will serve as the reporter probe. See the map below.

where ticks on scale are every 50 base pairs and ticks on restriction maps indicate where sites of each restriction enzyme are. The x marks indicate where the Ddel and MstII sites diagnostic of sickle cell anemia are located.

Each of the probes thus obtained is labeled either with biotin or fluoroscein using a psoralenated compound as described in U.S. Patent No. 4,582,789. The reactions are irradiated with UV light at 350-370 nm for 10 minutes and the reacted materials are isolated by ethanol precipitation.

Human genomic DNA homozygous for normal ß-globin is extracted from the lymphoid T cell line Molt 4 (Human Genetic Mutant Cell Repository (HMCR), Camden, N.J. GM2219C) and human genomic DNA homozygous for sickle cell anemia is extracted from EBV-transformed B cell line SC1 (American Type Culture Collection (ATCC), Rockville, MD, CRL 8756), using essentially the method of Maniatis et al.,supra, p. 280-281. Human genomic DNA from GM2064 (HMCR), which contains no beta- or delta-globin gene sequences, is extracted using essentially the method of Maniatis et al., supra, p. 280-281. Human genomic DNA donated from an individual with the heterozygous sickle cell allele is extracted from blood by the following procedure.

The DNA is most easily prepared from 10 to 50 ml of blood with separation from RBC's, lysing of white cells in high salt and detergent, extraction of nucleic acids using phenol, and an ethanol precipitation, as follows:

1) To the blood sample add 1/4 volume of 0.25 M Tris-HC1, pH 7.8, 0.5 M Na₂ EDTA, 1% Nonidet P-40. Mix gently but thoroughly.

2) Collect the crude preparation of nuclei by low-speed centrifugation in a conical tube. Suspend nuclei in 0.5 ml 0.05 M Tris, 0.1 M EDTA. Transfer to a 5 ml tube.

3) Add 0.5 ml 4 M NaCL, 2% Sarcosyl, and mix vigorously and thoroughly. Add 1 ml phenol/chloroform [1:1, phenol (saturated with Tris-EDTA) and chloroform], and emulsify. Continue vigorous shaking for five minutes, then centrifuge at high speed.

4) Collect the (upper) aqueous phase, leaving the organic phase and the band of denatured protein undisturbed. Transfer the solution of another 5 ml tube, then add 2.5 ml 95% ethanol. Mix thoroughly, then centrifuge at high speed.

5) Decant the supernatant, then add 3 ml 70% ethanol to tube. Mix vigorously (vortex) to suspend the pellet, then centrifuge at high speed. Decant the ethanol, then repeat the 70% ethanol wash. After centrifugation and decantation, add 3 ml 95% ethanol. Mix, then centrifuge at high speed. Decant the ethanol, then dry the tube.

6) Dissolve the pellet in 0.1 ml restriction enzyme digestion buffer, then add 0.01 ml restriction enzyme. Incubate at 37°C for one hour or more.

A ribonuclease plus amylase step can also be included and, to ensure the purity of DNA, nucleic acids can be centrifuged in a CsC1 buoyant density equilibrium gradient. The DNA is collected from gradient fractions by precipitation with three volumes of 70% ethanol, with the precipitate being washed with 70% ethanol and 95% ethanol. The precipitate is dried, the DNA is dissolved in TE (10 mM Tris.HC1, pH 7.6, 1 mM Na₂ EDTA) or water.

The DNA samples are digested with either Ddel or MstII or a combination of both according to the manufacturer's instructions. In using genomic DNA as the unknown, as will be the case in the best example and in the field, it is important that restriction enzyme digestions reach or closely approach completion. In particular, the Ddel/MstII site present in wild-type DNA, but absent in the sickle cell gene, has to be cleaved in samples of DNA lacking the sickle cell allele. Because MstII which is commercially available is not highly active, special care must be taken to ensure that digestions are as complete as possible. Such care can involve the use of an order of magnitude more enzyme than is theoretically required. It can also involve, in the special case of sickle cell, the digestion of DNA with excesses of both MstII and Ddel. Increasing the length of time of digestion should not be relied upon, since many enzymes become inactive within an hour or less of dilution

9

into a digestive mixture.

The genomic DNA is denatured by heating at 95°C for five minutes and rapidly cooling or by incubating with 0.2 M NaOH at 25°C for 10 minutes followed by exposure to 0.2 M HCl to give a pH of 6.5-8.5.

After cooling or neutralization, the two probes are added to each denatured DNA sample in a molar excess of 100,00 fold, and hybridization is carried out in 10mM phosphate buffer with o.5 M NaCl at 50-60°C for about three hours.

The reaction mixture is then applied to a microtiter well which has been pretereated with anti-fluorescein antibodies (MAB IgG2a to α-fluoroscein from Chemicon International Inc.), washed with uncoated antibodies, and pretreated with a mixture of PBS and herring sperm DNA as a blocking agent.

The reaction mixture is contacted with the pretreated plate by stirring or shaking for 15 minutes to 3 hours at room temperature, after which time the plate is washed with a wash solution of PBS and Triton X-100 detergent. After agitation for five minutes the rinse solvent is replaced with the wash solution containing a conjugate of avidin and horseradish peroxidase, obtainable commercially. The plate is contacted with the conjugate for 20 minutes, followed by rinsing with the wash solution to which is added urea. The plate is incubated with a mixture of sodium acetate, ethanol, 3,3',5,5'-tetramethylbenzidine (TMB) and $H_2O_2$.

A blue color appears for the SCI cell line, indicating that the $\underline{Mst}$II and the $\underline{Dde}$I restriction sites characteristic of normal ß-globin are not present in the cell line, which is correct. A blue color does not appear for the control GM2064 cell line, as expected, because there are no ß-globin sequences present. A blue color does not appear for the Molt 4 cell line, which has the $\underline{Mst}$II and $\underline{Dde}$I sites characteristic of normal ß-globin and is therefore cleaved. A blue color appears in the heterozygous sickle cell allele sample and can be separated from the homozygous case via spectral quantitation as the heterozygous sample should have one-half the signal for a given sample load.

One hour after incubation with TMB and $H_2O_2$, $H_2SO_4$ may be added to the reaction mixture. If the blue color has appeared, it will change to bright yellow, and the amount of the sequence present can be quantitated by measuring the absorbance of the reaction mixture at 450 $\lambda$.

The above process may be automated using a liquid handling instrument having microtiter wells as the solid support.

### EXAMPLE 2

A sequence contained in $\underline{N.\ gonorrheae}$ can be determined by constructing probes from the $\underline{N.\ gonorrheae}$ strain MSII pilus gene described by Meyer et al., $\underline{PNAS}$ (USA), 81:6110-6114 (1984). The pilus is a predominant surface antigen of $\underline{N.\ gonorrheae}$ and allows adhesion of the bacterium to host cells.

The probes may be constructed from DNA segments obtained by cleaving the MSII pilus gene with $\underline{Taq}$I. The segments to be used span positions 104-364 and 365-778 and are the contiguous 261 bp and 414 bp $\underline{Taq}$I fragments, respectively. See the map below

where ticks on scale are every 50 base pairs and ticks on $\underline{Taq}$I indicate where $\underline{Taq}$I sites are.

These fragments may be blunt-end ligated into M13 phage by standard cloning procedures as described in the above example and one resulting vector can be reacted with fluorescein and the other vector with biotin as described in the above example.

The procedure employed in Example 1 can then be utilized, except that no restriction enzyme is used to cleave the DNA after hybridization. If $\underline{N.\ gonorrheae}$ is present is the test sample, a blue color will appear, indicating that hybridization of the probes to the target has occurred.

In general, the invention provides the use, in a liquid hybridisation method for detecting a particular nucleic acid sequence, of at least two oligonucleotide probes, at least one of which probes is a reporter probe linked to the same or different label moieties capable of being detected, and at least one of which probes is a support-binding probe linked to a binding moiety which specifically recognizes a complementary binding molecule and which is different from the label moiety or moieties used for the reporter probe or probes, wherein all of said probes are complementary to different non-overlapping regions of the same strand of said nucleic acid sequence to be detected.

In summary the present invention is seen to provide a method for rapid and sensitive assay on nucleic acid sequences suspected of being contained in a sample. The assay process herein may be used to evaluate crude samples for rapid sample processing. Moreover, if both probes are specific, enhancement of specificity

0 238 332

for the nucleic acid sequence will occur. Binding to the solid support after hybridization ensures removal of interfering or background-generating material to yield a high signal to noise ratio. Furthermore, the process herein is relatively easy to automate and more reliable to manufacture, and no expense machines are needed to read the assay if an enzyme is used to detect the reporter probe(s). Enzyme use also results in greater sensitivity.

**Claims**

A liquid hybridization method for detecting whether a particular nucleic acid sequence suspected of being contained in a test sample of one or more nucleic acids is contained therein comprising the steps of:

(a) extracting the nucleic acids in the test sample;

(b) separating any extracted double-stranded nucleic acids into single strands;

(c) exposing the single-stranded nucleic acids to a molar excess of each of at least two oligonucleotide probes, at least one of which probes is a reporter probe linked to the same or different label moieties capable of being detected, and at least one of which probes is a support-binding probe linked to a binding moiety which specifically recognizes a complementary binding molecule and which is different from the label moiety or moieties used for the reporter probe or probes, wherein all of said probes are complementary to different non-overlapping regions of the same strand of said nucleic acid sequence to be detected;

(d) subjecting the mixture from step (c) to hybridization conditions favoring hybridization of said probes to said sequence;

(e) contacting the mixture of step (d) with a solid support phase which carries said complementary binding molecule specifically recognized by the binding moiety of said support-binding probe;

(f) washing the solid support phase to remove any unhybridized nucleic acids and cellular debris from the support phase; and

(g) detecting the presence or absence of said sequence by exposing the solid support to one or more means for detecting the label moiety or moieties of said reporter probe or probes.

2. A method according to claim 1 wherein said nucleic acids in said test sample are double-stranded DNA and said probes are oligodeoxyribonucleotide probes.

3. A method according to claim 1 or 2 wherein said probes are in a 10,000 to 1,000,000 fold molar excess over the test sample.

4. A method according to any one of claims 1-3 wherein the label moiety or moieties of the reporter probe or probes is or are detectable by spectroscopy or photochemistry or by formation of a detectable complex or complexes between the label moiety or moieties and a polypeptide, lectin or antibody having a detection means associated therewith.

5. A method according to any one of claims 1-4 wherein the sequence to be detected represents a restriction site, one probe is 3' to the site, one probe is 5' to the site, and neither probe overlaps the site, where if one probe spans a second sequence representing the same site, the probe which does not span any sequence representing the same site is the support-binding probe, and wherein after step (d) and before step (e) the mixture is contacted with a restriction enzyme which recognizes the restriction site to be detected.

6. A method according to claim 5 wherein the restriction site is characteristic of sickle cell anemia.

7. A method according to any one of claims 1-6 wherein the dissociation constant parameter for the binding reaction between the support-binding probe and the solid support phase in step (e) is less than $10^{-7}$ M.

8. A method according to any one of claims 1-7 wherein the solid support phase is treated prior to step (e) to block non-specific binding of the support-binding probe to the support phase.

9. A liquid hybridization method for detecting whether a particular DNA sequence suspected of being contained in a test sample of DNA is contained therein comprising the steps of:

(a) extracting the DNA in the test sample;

(b) separating the DNA into single strands;

(c) exposing the single-stranded DNA to a 10,000 to 1,000,000-fold molar excess of each of two DNA probes, one of which is a reporter probe labeled with biotin and complementary to one region of the DNA sequence to be detected, and one of which is a support-binding probe attached to fluorescein and complementary to a second region of said sequence, said two regions being from the same strand of the DNA sequence to be detected and being non-overlapping;

(d) subjecting the mixture from step (c) to hybridization conditions favoring hybridization of said probes to said DNA sequence;

(e) contacting the mixture of step (d) with a solid support phase which carries antibodies which specifically bind to fluorescein and which has been pre-treated to block non-specific binding of the support-binding probe to the support phase;

(f) washing the solid support phase to remove any unhybridized nucleic acids and cellular debris

11

from the solid support phase;

(g) adding an avidin-enzyme or streptavidin-enzyme conjugate to the washed solid support; and

(h) adding a substrate for the enzyme to the enzyme-treated support and using spectroscopic means to detect the formation of the product of the enzyme-catalyzed reaction.

10. A liquid hybridization assay kit for determining whether a particular nucleic acid sequence suspected of being contained in a test sample of one or more nucleic acids is contained therein comprising, in packaged multi-container format,

(a) container(s) for at least one oligonucleotide reporter probe which is linked to a label moiety capable of being detected and which probe is complementary to a first region of one strand of the nucleic acid sequence to be detected;

(b) a container for an oligonucleotide support-binding probe which is linked to a binding moiety which is different from said first label moiety and which specifically recognizes a complementary binding molecule, wherein the support-binding probe is complementary to a second region of the same strand of said sequence which region does not overlap the first region of said sequence; and

(c) a solid support carrying said complementary binding molecule which is specifically recognized by the binding moiety of the support-binding probe.

11. The use, in a liquid hybridization method for detecting a particular nucleic acid sequence, of at least two oligonucleotide probes, at least one of which probes is a reporter probe linked to the same or different label moieties capable of being detected, and at least one of which is a support-binding probe linked to a binding moiety which specifically recognizes a complementary binding molecule and which is different from the label moiety or moieties used for the reporter probe or probes, wherein all of said probes are complementary to different non-overlapping regions of the same strand of said nucleic acid sequence to be detected.